# EUROPEAN PATENT SPECIFICATION

(11) **EP 3 765 018 B1**
(45) Date of publication and mention of the grant of the patent: **29.04.2026**
(21) Application number: 19766563.1
(22) Date of filing: 13.03.2019
(51) Int. Cl.: A61K 31/4738, A61K 31/7084, A61K 31/716, A61K 39/00, A61P 35/00

(54) **BETA GLUCAN AND CD40 AGONIST COMBINATION IMMUNOTHERAPY FOR TREATING PANCREATIC CANCER**
BETA-GLUCAN UND CD40-AGONIST-KOMBINATIONSIMMUNTHERAPIE ZUR BEHANDLUNG VON BAUCHSPEICHELDRÜSENKREBS
IMMUNOTHÉRAPIE ASSOCIANT UN BÊTA-GLUCANE ET UN AGONISTE DE CD40 POUR LE TRAITEMENT DU CANCER DU PANCREAS

(30) Priority: 13.03.2018 US 201862642210 P
(43) Date of publication of application: 20.01.2021
(73) Proprietor: Hibercell, Inc., New York, NY 10019 (US); The Trustees of The University of Pennsylvania, Philadelphia, PA 19104 (US)
(72) Inventor: BOSE, Nandita, Plymouth, Minnesota 55446 (US); BEATTY, Gregory, Philadelphia, Pennsylvania 19106 (US)
(74) Representative: Kilburn & Strode LLP
(86) International application number: PCT/US2019/022062
(87) International publication number: WO 2019/178236

(56) References cited:
- WO-A1-2016/023960
- WO-A2-2007/084661
- US-A1- 2017 100 425
- US-A1- 2017 298 139
- US-A1- 2017 369 570
- US-B2- 9 522 187
- BEATTY GREGORY L ET AL: "CD40 Agonists Alter Tumor Stroma and Show Efficacy Against Pancreatic Carcinoma in Mice and Humans", SCIENCE, vol. 331, no. 6024, 25 March 2011 (2011-03-25), US, pages 1612 - 1616, XP055825709, ISSN: 0036-8075, DOI: 10.1126/science.1201079

## Description

### SUMMARY

This disclosure provides, in one aspect, compositions of soluble β-glucan and a CD40 agonist and their use for cancer immunotherapy. The CD40 agonist may be an agonistic CD40 antibody and the soluble β-glucan may be derived from yeast.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

The aspects of the invention for which protection is sought are set out in the appended set of claims.

The technical information set out below may in some respects go beyond the scope of the present invention, which is defined by the appended set of claims. The additional technical information is provided to place the present invention in a broader technical context and to illustrate possible related technical developments.

In one aspect, the invention provides soluble β-glucan for use in a method of treatment of pancreatic cancer, the method comprising administering the soluble β-glucan and an agonistic CD40 antibody, wherein the soluble β-glucan is β(1,6)-[poly-(1,3)-D-glucopyranosyl]-poly-β(1,3)-D-glucopyranose.

In a further aspect, the invention provides an agonistic CD40 antibody for use in a method of treatment of pancreatic cancer, the method comprising administering the agonistic CD40 antibody and soluble β-glucan, wherein the soluble β-glucan is β(1,6)-[poly-(1,3)-D-glucopyranosyl]-poly-β(1,3)-D-glucopyranose.

In a further aspect, the invention provides soluble β-glucan and an agonistic CD40 antibody for use in a method of treatment of pancreatic cancer, the method comprising co-administering simultaneously or sequentially the soluble β-glucan and the agonistic CD40 antibody, wherein the soluble β-glucan is β(1,6)-[poly-(1,3)-D-glucopyranosyl]-poly-β(1,3)-D-glucopyranose.

Preferred features of the invention are set out in the dependent claims herein.

The references to methods of treatment in the subsequent paragraphs of this description are to be interpreted as references to the compounds, pharmaceutical compositions and medicaments of the present invention for use in a method for treatment of the human (or animal) body by therapy.

### BRIEF DESCRIPTION OF THE FIGURES

Figures 1 A-1E. Tumor growth curves for treatment combinations including soluble β-glucan and FGK45.
Figures 2A-2E. Tumor growth curves for treatment combinations including soluble β-glucan and Gemcitabine chemotherapy.
Figures 3A-3B. Graphs comparing tumor growth between treatment groups.
Figure 4. Graph comparing overall survival between treatment groups.
Figure 5 A. Graph comparing response between treatment groups.
Figure 5B. Graph comparing overall survival between treatment groups.
Figures 5C-5E. Graphs comparing pharmacodynamic changes between treatment groups.

### DETAILED DESCRIPTION OF ILLUSTRATIVE EMBODIMENTS

Drugs designed to harness the immune system to treat cancer have shown promising results but, when used individually, many patients do not respond to these drugs or responses are transient. Thus, certain immunotherapeutic drugs may be combined to bridge the innate and adaptive arms of the immune system and drive enhanced cancer therapy. Specifically, the present invention, as defined by the appended set of claims, relates to compositions of β-glucan and CD40 agonists and their use in cancer immunotherapy.

β-glucans are polymers of glucose derived from a variety of microbiological and plant sources including, for example, yeast, bacteria, algae, seaweed, mushroom, oats, and barley. Of these, yeast β-glucans have been extensively evaluated for their immunomodulatory properties. Yeast β-glucans can be present as various forms such as, for example, intact yeast, zymosan, purified whole glucan particles, solubilized zymosan polysaccharide, or highly-purified soluble β-glucans of different molecular weights. Structurally, yeast β-glucans are composed of glucose monomers organized as a β-(1,3)-linked glucopyranose backbone with periodic β-(1,3) glucopyranose branches linked to the backbone via β-(1,6) glycosidic linkages. The different forms of yeast β-glucans can function differently from one another. The mechanism through which yeast β-glucans exert their immunomodulatory effects can be influenced by the structural differences between different forms of the β-glucans such as, for example, its particulate or soluble nature, tertiary conformation, length of the main chain, length of the side chain, and frequency of the side chains. The immune stimulating functions of yeast β-glucans are also dependent upon the receptors engaged in different cell types in different species, which again, can be dependent on the structural properties of the β-glucans.

In general, β-glucan immunotherapies can include administering to a subject any suitable form of β-glucan or any combination of two or more forms of β-glucan. Suitable β-glucans and the preparation of suitable β-glucans from their natural sources are described in, for example, U.S. Patent No. 9,610,303. In some cases, the β-glucan may be derived from a yeast such as, for example, *Saccharomyces cerevisiae.* According to the present invention, the β-glucan is β(1,6)-[poly-(1,3)-D-glucopyranosyl]-poly-β(1,3)-D-glucopyranose, also referred to herein as PGG (IMPRIME PGG, Biothera, Eagan, MN), a highly purified and well characterized form of soluble yeast-derived β-glucan. β-glucan is a PAMP (pathogen-associated molecular pattern) capable of triggering innate immune cell function leading to a cascade of immune activation and enhanced anti-tumor killing. Yeast soluble β-glucan has been known to enhance the direct killing of tumor cells by innate immune effector cells in the presence of tumor-targeting antibodies. However, yeast soluble β-glucan also enhances T cell-based cancer therapies through two mechanisms. Specifically, yeast soluble β-glucan triggers re-polarization of myeloid function to counteract the immune suppressive microenvironment established by a tumor. In addition, yeast soluble β-glucan activates the maturation of antigen presenting cells (i.e., macrophages and dendritic cells) enabling T cell expansion and activation as well as expression of IFN-y. Unlike other PAMPs where systemic administration often leads to toxic side effects, Imprime PGG has been administered safely by intravenous infusion to more than 400 human subjects. Moreover, β-glucan-based immunotherapies can involve the use of, for example, a modified and/or derivatized β-glucan such as those described in International Patent Application No. PCT/US12/36795. In other cases, β-glucan immunotherapy can involve administering, for example, a particulate-soluble β-glucan or a particulate-soluble β-glucan preparation, each of which is described in, for example, U.S. Patent No. 7,981,447.

Biomarker research demonstrated differences among subjects in the ability of their neutrophils and monocytes to bind yeast soluble β-glucan. Binding of yeast soluble β-glucan to these cells correlated with the subjects' immunomodulatory response to yeast soluble β-glucan. Moreover, yeast soluble β-glucan binding to neutrophils and monocytes involves the presence of a specific level of natural anti-β-glucan antibodies. Biomarker assay methods to quantitatively measure anti-β-glucan IgG and IgM antibodies (ABAs) in patient serum samples are described in International Published Application Nos. WO2013165591A1 and WO2015084732A1. Cutoff levels for the biomarker assay stratify subjects into biomarker positive and biomarker negative subgroups and these cutoff points correlate with binding, function, and clinical outcomes.

If a subject is identified as biomarker negative because the subject's level of ABAs falls below a selected, predetermined cutoff level, an antibody preparation capable of converting the subject from biomarker negative status to biomarker positive status can be administered to improve the subject's response to the immunotherapy. Thus, in some cases, the β-glucan and CD40 agonist combination immunotherapies may also include administration of an antibody preparation to improve the subject's response to the immunotherapy. Compositions and methods for improving a subject's response to immunotherapy are described in International Published Application No. WO2013165593A1.

In some cases, the antibody preparation can include serum from a biomarker positive subject. In some cases, the antibody preparation can include a monoclonal antibody or antibody fragment that specifically binds the β-glucan. In some cases, the antibody preparation can include intravenous immunoglobulin.

WO 2007/084661 A2 relates to compositions comprising an effective amount of a yeast beta-glucan composition which is suitable for oral administration and for absorption through the gastrointestinal tract of a mammal for the treatment of cancer.

US 9,522,187 B2 relates to methods of using neutral soluble glucan and monoclonal antibodies for antitumor therapy.

Beatty et al., (2011), Science:331 describes that CD40 agonists alter tumor stroma and show efficacy against pancreatic carcinoma in mice and humans.

US 2017100425 A1 relates to the combination of soluble β-glucan and anti-cancer agents that affect the tumor microenvironment.

The CD40 molecule is a member of the TNF receptor superfamily and is expressed by multiple cell types including monocytes, macrophages, dendritic cells, B cells, platelets, fibroblasts, endothelial cells, smooth muscle cells and some malignant epithelial cells. The ligand for CD40, CD40 ligand (CD 154), is expressed on a variety of cells including activated CD4 T cells, activated B cells, memory CD8 T cells, activated natural killer cells, granulocytes, endothelial cells, smooth muscle cells, and activated platelets. Activation of the CD40 pathway is a critical step in 'licensing' antigen presenting cells with the capacity to effectively present antigen and stimulate antigen-specific T cells. Ligation of CD40 on dendritic cells (DCs) enhanced the expression of costimulatory (e.g. CD80 and CD86) and major histocompatibility (MHC) molecules, induced the release of immunostimulatory cytokines and activated antigen presentation machinery. The importance of CD40 in tumor immunity was subsequently demonstrated in several studies where administration of an agonistic antibody directed against CD40 produced protective T cell immunity in murine models of cancer. This was the basis for the development of clinical grade CD40 agonists that are currently in clinic studies.

The CD40 agonist useful in the present invention may be a recombinant human CD40 ligand such as Avrend (Immunex Corp, Seattle, WA), CD40 ligand gene therapy such as an adenoviral vector serotype 5 carrying the human *CD40L* gene driven by a Rous sarcoma virus promoter (AdCD40L) (Vector Production Facility at the Center for Cell and Gene Therapy, Baylor College of Medicine, Houston, TX) or agonistic CD40 antibodies. Agonistic CD40 antibodies may be polyclonal antibodies, antibody fragments or monoclonal antibodies of various isotypes such as, for example, fully human IgG2, humanized rabbit IgGl, fully human IgGl, chimeric IgG1 and non-fucosylated humanized IgG1 isotypes.

Agonistic CD40 antibodies having an IgG1 Fc domain may be beneficial in some cases, because the IgG1 Fc domain enhances binding to Fcγ receptor II-B (FcγRIIB), which potentially increases CD40 agonist potency through crosslinking. Alternatively, agonistic CD40 antibodies may be chemically crosslinked for Fc-independent enhanced activity. In other cases, agonistic CD40 antibodies with an IgG2 Fc domain may mediate FcγR-independent agonistic activity through the unique hinge properties of the IgG2 isotype. Alternatively, in some cases, neither the Fc domain of the antibody nor Fc receptor crosslinking are required for CD40 stimulation by agonistic CD40 antibodies. The precise mechanism underlying this finding is unclear but could be explained by certain Fab regions binding to a specific epitope on human CD40 that produces potent signaling activity.

FcγRIIB is the only inhibitory Fc receptor and variations in the gene encoding this protein, or polymorphisms, have long been associated with susceptibility to autoimmune disease. These polymorphisms could have therapeutic implications related to the efficacy of IgGl-modified CD40 agonists such that various FcγRIIB polymorphisms would correlate with administration of specific CD40 antibodies. For example, subjects with FcγRIIB polymorphisms correlating with reduced efficacy of IgGl-modified CD40 agonists would only be administered IgG2 CD40 agonists or agonistic CD40 antibodies lacking the Fc domain.

In addition, in murine models, the enhanced potency of IgG1 CD40 agonists is associated with an increased risk of transient thrombocytopenia. Reducing the dose of the IgG1 CD40 agonist diminished the level of thrombocytopenia while still maintaining improved agonist activity compared to the IgG2 isotype. Thus, in some cases, a reduced dose or less frequent dosing of an IgG1 CD40 agonist may be preferred for the β-glucan and agonistic CD40 antibody combined immunotherapy.

Several agonistic CD40 monoclonal antibodies have been developed for clinical use that may be used in the present invention. Each antibody is distinct with unique properties defined by (i) binding affinity to CD40, (ii) isotype, (iii) dependence on crosslinking for activity and (iv) the ability to block CD40 ligand binding. These include:
(1) **CP-870,893** (Pfizer and VLST) is an anti-CD40 IgG2 antibody with poor FcR binding that does not block CD40 ligand interaction with CD40; can mediate CD40 stimulation in the absence of cross-linking; and has a binding affinity (Kd) of 3.48 x 10⁻¹⁰M.
(2) **APX005** (Apexigen) is an IgG1 antibody recognizing CD40 that blocks CD40 ligand binding; shows enhanced activity *in vitro* with cross-linking; and has a Kd of 9.6 x 10⁻¹⁰M.
(3) **ADC-1013** (Alligator Bioscience) is an IgG1 antibody that recognizes CD40 with high binding affinity (Kd 1 x 10⁻¹¹M) even under acidic conditions (pH 5.4) with activity dependent on FcR binding and crosslinking.
(4) **Dacetuzumab (also called SGN-40 and formerly SGN-14)** (Seattle Genetics) is a humanized IgG1 antibody recognizing CD40 with a Kd of 1 x 10⁻⁹M. Dacetuzumab is a partial agonist that shows weak activity in stimulating B cell proliferation; displays potent antiproliferative and pro-apoptotic properties against B cell lymphoma lines and enhances CD40 ligand binding to CD40.
(5) **SEA-CD40** (Seattle Genetics) is a non-fucosylated humanized IgG1 anti-CD40 antibody derived from dacetuzumab (SGN-40) with a Kd of 1 x 10⁻⁹M. SEA-CD40 shows improved agonist activity due to enhanced binding to FcgRIIIa.
(6) **ChiLob 7/4** (University of Southamptom) is a chimeric IgG1 antibody with a Kd of 2x10⁻¹⁰M that requires crosslinking for CD40 stimulation in antigen-presenting cells.

Poor responsiveness to immunotherapy that is seen in many patients may be due to the absence of an existing ongoing immune response against the cancer that is necessary for immunotherapy to be successful. As such, the present invention includes novel approaches, such as the use of immune agonists, capable of invoking potent antitumor immunity to broaden and enhance the therapeutic impact of cancer immunotherapy.

The efficacy of CD40 agonists has been reported to be dependent upon the presence of tumor antigen, which is necessary for CD40-activated antigen-presenting cells to induce antigen- specific T cell immunity. Subsequent studies were then focused on combining CD40 agonists with vaccines and chemotherapies to provoke an immunogenic form of tumor cell death. However, even in combination with chemotherapy, evidence for T cell-dependent antitumor activity was not observed and further studies identified immunosuppressive macrophages in the tumor microenvironment. These macrophages in addition to other immunosuppressive mechanisms likely explain the lack of T cell-dependent antitumor activity.

Soluble β-glucan binds to CD11b on cells of myeloid origin, namely neutrophils and monocytes, and increases the immunostimulatory functions of Nl neutrophils and Ml macrophages and decreases the immunosuppressive functions of myeloid-derived suppressor cells, N2 neutrophils and M2 macrophages. This modulation results in cross-talk between the various innate and adaptive cells in the tumor microenvironment, which redirects conditions toward immunostimulation. Soluble β-glucan directly affects M2 repolarization to the Ml phenotype and drives Thl polarization, and soluble β-glucan-primed innate immune cells generate cytokines to indirectly affect CD4 and CD8 T cell proliferation, even in the presence of T-regulatory cells, and eventually drive Thl polarization.

To this end, soluble β-glucan can be combined with CD40 agonists. For example, soluble β-glucan can be combined with agonistic CD40 antibodies in the treatment of cancers including, for example, melanoma, pancreatic cancer, multiple myeloma, non-Hodgkin's lymphoma, chronic lymphocytic leukemia, mesothelioma, advanced solid tumors, etc. Soluble β-glucan triggers re-polarization of myeloid function to counteract the immune suppressive microenvironment established by a tumor and activates the maturation of antigen-presenting cells leading to T cell expansion and activation and IFN-γ expression. These conditions are ideal for CD40 agonists to induce T cell-dependent antitumor immunity. The combination of a CD40 agonist and soluble β-glucan results in a synergistic effect that greatly enhances therapeutic outcomes even against poorly immunogenic tumors.

The invention includes, in part, co-administering a soluble β-glucan with a CD40 agonist in accordance with appended claim 3. As used herein, "co-administered" refers to two or more components of a combination administered so that the therapeutic or prophylactic effects of the combination can be greater than the therapeutic or prophylactic effects of either component administered alone. Two components may be co-administered simultaneously or sequentially. Simultaneously co-administered components may be provided in one or more pharmaceutical compositions. Sequential co administration of two or more components is independent of timing and includes cases in which the components are administered so that both components are simultaneously bioavailable after both are administered. Regardless of whether the components are co-administered simultaneously or sequentially, the components may be co-administered at a single site or at different sites.

The soluble β-glucan, the CD40 agonist, and/or the combination of both components for use according to the invention may be formulated in a composition along with a "carrier." As used herein, "carrier" includes any solvent, dispersion medium, vehicle, coating, diluent, antibacterial, and/or antifungal agent, isotonic agent, absorption delaying agent, buffer, carrier solution, suspension, colloid, and the like. The use of such media and/or agents for pharmaceutically active substances is well known in the art. Except insofar as any conventional media or agent is incompatible with the β-glucan or the CD40 agonist, its use in the therapeutic compositions is contemplated. Supplementary active ingredients also can be incorporated into the compositions. By "pharmaceutically acceptable" is meant a material that is not biologically or otherwise undesirable, i.e., the material may be administered to an individual along with the β-glucan and/or the pharmaceutical agent without causing any undesirable biological effects or interacting in a deleterious manner with any of the other components of the pharmaceutical composition in which it is contained.

The soluble β-glucan, the CD40 agonist, and/or the combination of both components for use according to the invention may be formulated into a pharmaceutical composition. In some embodiments, the soluble β-glucan and the CD40 agonist may be provided in a single formulation. In other embodiments, the soluble β-glucan and the CD40 agonist may be provided in separate formulations. A pharmaceutical composition comprising the soluble β-glucan, the CD40 agonist, and/or the combination of both components for use according to the invention may be formulated in a variety of and/or a plurality forms adapted to one or more preferred routes of administration. Thus, a pharmaceutical composition comprising the soluble β-glucan, the CD40 agonist, and/or the combination of both components for use according to the invention can be administered via one or more known routes including, for example, oral, parenteral (e.g., intradermal, transcutaneous, subcutaneous, intramuscular, intravenous, intraperitoneal, intratumoral, etc.), or topical (e.g., intranasal, intrapulmonary, intramammary, intravaginal, intrauterine, intradermal, transcutaneous, rectally, etc.). A pharmaceutical composition, or a portion thereof, comprising the soluble β-glucan, the CD40 agonist, and/or the combination of both components for use according to the invention can be administered to a mucosal surface, such as by administration to, for example, the nasal or respiratory mucosa (e.g., by spray or aerosol). A pharmaceutical composition, or a portion thereof, comprising the soluble β-glucan, the CD40 agonist, and/or the combination of both components for use according to the invention also can be administered via a sustained or delayed release.

A formulation of the soluble β-glucan, the CD40 agonist, and/or the combination of both components for use according to the invention may be conveniently presented in unit dosage form and may be prepared by methods well known in the art of pharmacy. As part of the disclosure but not the invention methods of preparing a composition with a pharmaceutically acceptable carrier for use according to the invention include the step of bringing the β-glucan and/or the Cd40 agonist into association with a carrier that constitutes one or more accessory ingredients. As part of the disclosure but not the invention, a formulation for use according to the invention may be prepared by uniformly and/or intimately bringing the active compound into association with a liquid carrier, a finely divided solid carrier, or both, and then, if necessary, shaping the product into the desired formulations.

The soluble β-glucan, the CD40 agonist, and/or the combination of both components for use according to the invention may be provided in any suitable form including but not limited to a solution, a suspension, an emulsion, a spray, an aerosol, or any form of mixture. A composition for use according to the invention may be delivered in formulation with any pharmaceutically acceptable excipient, carrier, or vehicle. For example, a formulation for use according to the invention may be delivered in a conventional topical dosage form such as, for example, a cream, an ointment, an aerosol formulation, a non-aerosol spray, a gel, a lotion, and the like. The formulation for use according to the invention may further include one or more additives including such as, for example, an adjuvant, a skin penetration enhancer, a colorant, a fragrance, a flavoring, a moisturizer, a thickener, and the like.

In some embodiments, the invention can include administering sufficient soluble β-glucan to provide a dose of, for example, from about 100 ng/kg to about 50 mg/kg to the subject, although in some embodiments the soluble β-glucan may be administered in a dose outside this range. The dose may be dependent on a subject's ABA levels. In some embodiments, the β-glucan dose range is about 0.5 mg/kg to about 6 mg/kg including 0.5 mg/kg, 1.0 mg/kg, 1.5 mg/kg, 2.0 mg/kg, 2.5 mg/kg, 3.0 mg/kg, 3.5 mg/kg, 4.0 mg/kg, 4.5 mg/kg, 5.0 mg/kg, 5.5 mg/kg and 6.0 mg/kg. The soluble β-glucan may only be dosed once or time intervals between dosing may be, for example, every 1, 2, 3, 4, 5, 6, 7, 8, 9, 10, 11 or 12 weeks during a course of treatment or every 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83 or 84 days. The dosing schedule will vary depending on, among other things, the cancer being treated and the subject's ABA level. Soluble β-glucan dosing is described in International Patent Application No. PCT/US18/19412.

Alternatively, the dose may be calculated using actual body weight obtained just prior to the beginning of a treatment course. For the dosages calculated in this way, body surface area (m²) is calculated prior to the beginning of the treatment course using the Dubois method: m² = (wt kg^{0.425} x height cm^{0.725}) x 0.007184. In some embodiments, therefore, the method can include administering sufficient soluble β-glucan to provide a dose of, for example, from about 0.01 mg/m² to about 10 mg/m².

The invention includes administering sufficient CD40 agonist. The dose of CD40 agonist may be from 0.01 mg/kg to 20 mg/kg, including 0.01 mg/kg, 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.5 mg/kg, 2.0 mg/kg, 2.5 mg/kg, 3.0 mg/kg, 3.5 mg/kg, 4.0 mg/kg, 4.5 mg/kg, 5.0 mg/kg, 5.5 mg/kg, 6.0 mg/kg, 6.5 mg/kg, 7.0 mg/kg, 7.5 mg/kg, 8.0 mg/kg, 8.5 mg/kg, 9.0 mg/kg, 9.5 mg/kg, 10.0 mg/kg, 10.5 mg/kg, 11.0 mg/kg, 11.5 mg/kg, 12.0 mg/kg, 12.5 mg/kg, 13.0 mg/kg, 13.5 mg/kg, 14.0 mg/kg, 14.5 mg/kg, 15.0 mg/kg, 15.5 mg/kg, 16.0 mg/kg, 16.5 mg/kg, 17.0 mg/kg, 17.5 mg/kg, 18.0 mg/kg, 18.5 mg/kg, 19.0 mg/kg, 19.5 mg/kg and 20.0 mg/kg. The CD40 agonist may only be dosed once or time intervals between dosing may be, for example, every 1, 2, 3, 4, 5, 6, 7, 8, 9, 10, 11 or 12 weeks during a course of treatment or every 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83 or 84 days.

Alternatively, the dose of either soluble β-glucan or CD40 agonist may be calculated using actual body weight obtained just prior to the beginning of a treatment course. For the dosages calculated in this way, body surface area (m²) is calculated prior to the beginning of the treatment course using the Dubois method: m² = (wt kg^{0.425} x height cm^{0.725}) x 0.007184. In some embodiments, therefore, the method can include administering sufficient β-glucan and/or CD40 agonist to provide a dose of, for example, from about 0.01 mg/m² to about 10 mg/m².

The present invention is illustrated by the following examples.

### Example 1

**Soluble β-glucan in combination with a CD40 agonist enhances tumor regression:** The role for soluble β-glucan in inducing potent anti-tumor immunity against pancreatic ductal adenocarcinoma (PDAC), a poorly immunogenic cancer that is marked by T cell exclusion and a robust inflammatory immune reaction, was evaluated. In this study, C57BL/6 mice were implanted subcutaneously with a KPC-derived PDAC cell line (7940B, P9). Mice were monitored 2-3 times per week for tumor growth by calipers. On day -2, mice received gemcitabine (120 mg/kg) or PBS. On day 0, mice were treated with or without FGK45 (0.1 mg), IMPRIME PGG (1.2 mg), or both. FGK45 is a rat anti-mouse monoclonal agonistic antibody to CD40 and was administered by intraperitoneal injection. IMPRIME PGG was administered by intravenous injection.

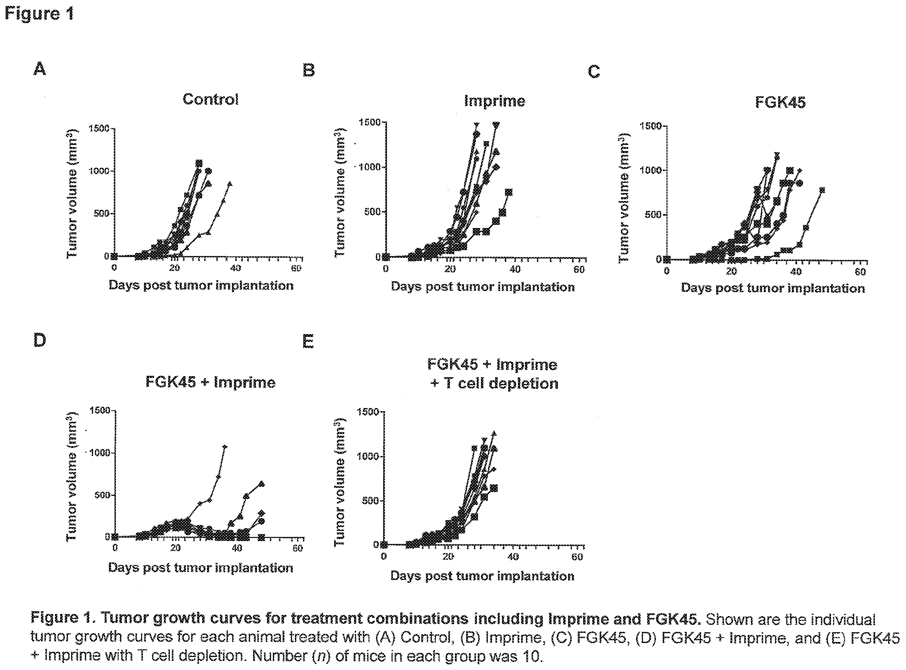

Mice were followed for tumor growth and overall survival (i.e. until tumors reached >1000 mm³ or animals demonstrated tumor-related discomfort/distress). The impact of IMPRIME PGG treatment alone on tumor growth versus the control is shown in Figures 1A and 1B. Here, treatment with a single dose of IMPRIME PGG produced a delay in tumor outgrowth in 1 of 10 mice. In a previous experiment, IMPRIME PGG produced a delay in tumor outgrowth in 3 of 9 mice (data not shown). These experiments indicate that IMPRIME PGG has limited activity as monotherapy in this tumor model. Similar results, shown in Figure 1C, were seen with FGK45 treatment alone where delayed tumor growth was seen in 4 of 10 mice. A previous experiment showed delayed tumor growth in 5 of 9 mice (data not shown).

In contrast, combining IMPRIME PGG and FGK45 produced tumor regressions in 9 of 10 mice (Figure 1D). Complete tumor regressions were seen in 6 of 10 mice without subsequent recurrence.

### Example 2

**Soluble β-glucan in combination with an agonistic CD40 antibody invokes T cell-dependent antitumor activity against a poorly immunogenic tumor:** On days -3, 0, 4, 7 and 11, mice from Example 1 were treated with or without GK1.5 (anti-CD4) and 2.43 (anti-CD8) depleting antibodies (0.2 mg/dose). 2.43 and GK1.5 are rat anti-mouse monoclonal antibodies that deplete CD8α and CD4 expressing T cells, respectively. Both antibodies were administered by intraperitoneal injection.

As shown in Figure 1E, the synergistic therapeutic activity using the combination of IMPRIME PGG and FGK45 was ablated when CD4 and CD8 T cells were depleted. Thus, this data shows that combining IMPRIME PGG and FGK45 produces remarkable T cell-dependent anti-tumor activity in a poorly immunogenic model of murine pancreatic ductal adenocarcinoma.

### Example 3

**Soluble β-glucan in combination with chemotherapy enhances tumor regression:** C57BL/6 mice were implanted subcutaneously with a KPC-derived PDAC cell line (7940B, P9). Mice were monitored 2-3 times per week for tumor growth by calipers. On day -2, mice received gemcitabine (120 mg/kg) or PBS.

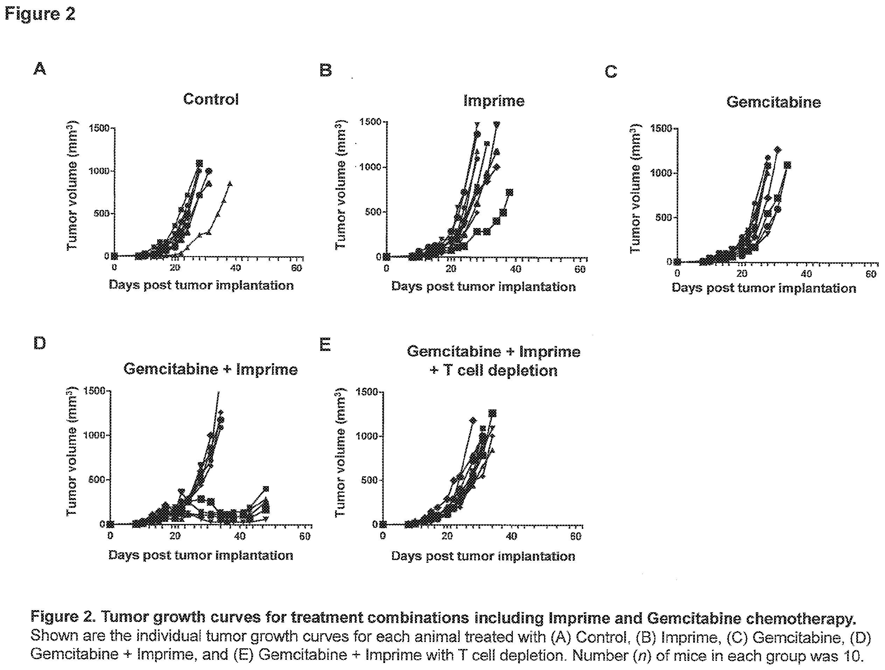

Again, IMPRIME PGG alone demonstrated minimal activity (Figure 2A, B). Similarly, as shown in Figure 2C, gemcitabine chemotherapy produced no significant impact on tumor growth. In contrast, treatment with gemcitabine chemotherapy administered 48 hours prior to IMPRIME PGG produced significant anti-tumor activity with delayed tumor outgrowth seen in 5 of 10 mice (Figure 2D). In a previous experiment, this treatment combination produced anti-tumor activity in 6 of 9 mice.

### Example 4

**Soluble β-glucan in combination with chemotherapy invokes T cell-dependent antitumor activity against a poorly immunogenic tumor:** On days -3, 0, 4, 7 and 11, mice from Example 3 were treated with or without GK1.5 (anti-CD4) and 2.43 (anti-CD8) depleting antibodies (0.2 mg/dose). The synergistic therapeutic effect using the combination of IMPRIME PGG and gemcitabine is dependent on CD4 and CD8 T cells as seen when T cells are depleted prior to treatment (Figure 2E). Thus, combining chemotherapy with IMPRIME PGG can produce significant T cell dependent anti-tumor activity in a poorly immunogenic model of murine pancreatic ductal adenocarcinoma.

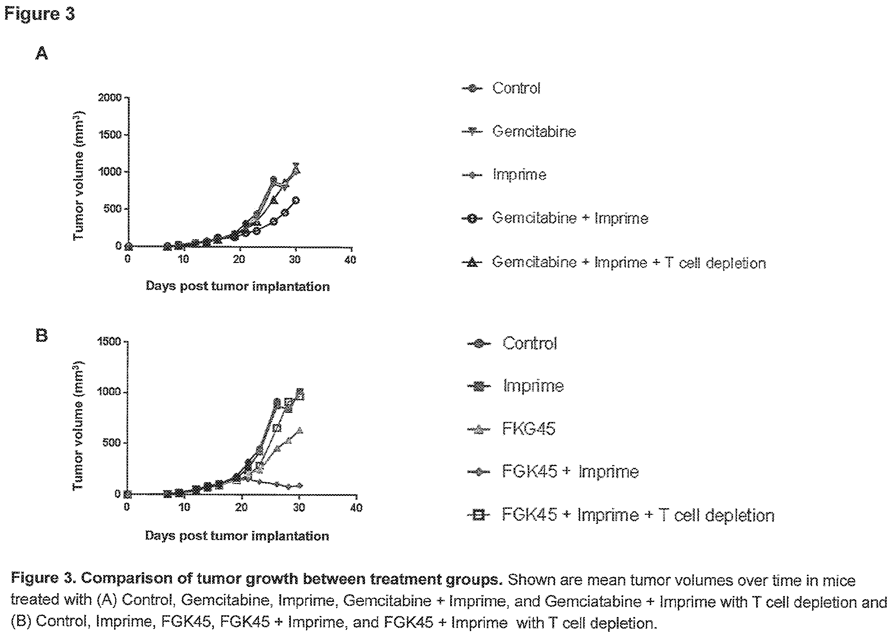

Figures 3A and 3B show the mean tumor growth curves for each gemcitabine and FGK45 treatment group, respectively. Statistical significance for tumor growth comparisons at day 26 was determined using two-way Anova with Tukey's multiple comparisons testing. Statistics between treatment groups are shown in Table 1.

**Table 1**

| **Treatment Compared** | ***P* Value** |
|---|---|
| Ctrl vs IMPRIME | 0.9944 |
| Ctrl vs FGK45 | <0.0001 |
| Ctrl vs Gemcitabine | 0.9750 |
| Ctrl vs FGK45+IMPRIME | <0.0001 |
| Ctrl vs Gemcitabine+IMPRIME | <0.0001 |
| IMPRIME vs FGK45 | <0.0001 |
| IMPRIME vs Gemcitabine | >0.9999 |
| IMPRIME vs FGK45+IMPRIME | <0.0001 |
| IMPRIME vs Gemcitabine+IMPRIME | <0.0001 |
| FGK45 vs Gemcitabine | 0.0001 |
| FGK45 vs FGK45+IMPRIME | 0.0008 |
| FGK45 vs Gemcitabine+IMPRIME | 0.7767 |
| Gemcitabine vs FGK45+IMPRIME | <0.0001 |
| Gemcitabine vs Gemcitabine+IMPRIME | <0.0001 |
| FGK45+IMPRIME vs Gemcitabine+IMPRIME | 0.0601 |

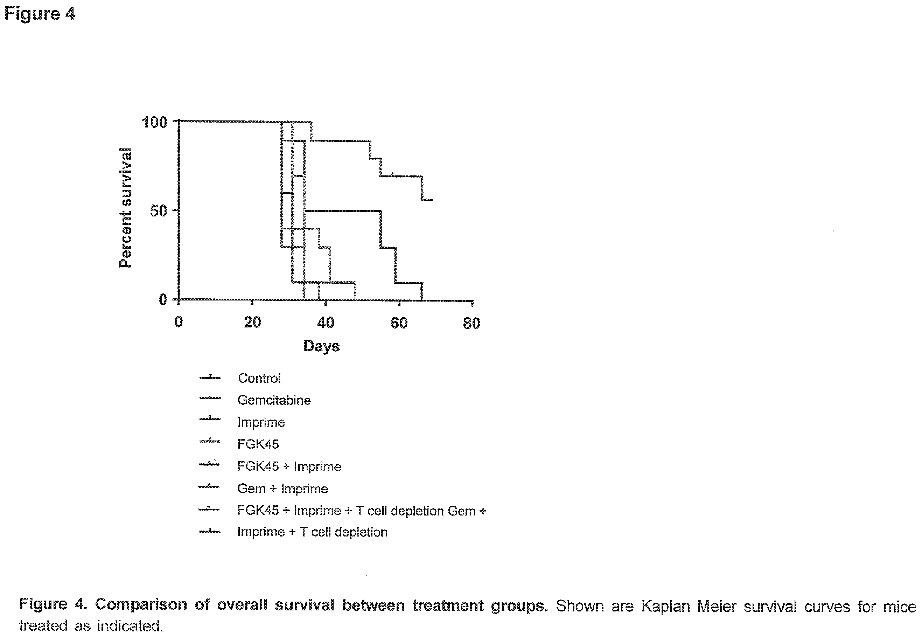

Figure 4 shows the impact of each treatment on overall survival. Statistics for survival comparisons between treatment groups are shown in Table 2. Statistical significance was determined using Log Rank test.

**Table 2**

| **Treatment Compared** | ***P* Value** |
|---|---|
| Ctrl vs IMPRIME | 0.2794 |
| Ctrl vs FGK45 | 0.0024 |
| Ctrl vs Gemcitabine | 0.7345 |
| Ctrl vs FGK45+IMPRIME | <0.0001 |
| Ctrl vs Gemcitabine+IMPRIME | 0.0002 |
| IMPRIME vs FGK45 | 0.0291 |
| IMPRIME vs Gemcitabine | 0.3989 |
| IMPRIME vs FGK45+IMPRIME | <0.0001 |
| IMPRIME vs Gemcitabine+IMPRIME | 0.0029 |
| FGK45 vs Gemcitabine | 0.0074 |
| FGK45 vs FGK45+IMPRIME | <0.0001 |
| FGK45 vs Gemcitabine+IMPRIME | 0.0284 |
| Gemcitabine vs FGK45+IMPRIME | <0.0001 |
| Gemcitabine vs Gemcitabine+IMPRIME | 0.0012 |
| FGK45+IMPRIME vs Gemcitabine+IMPRIME | 0.0048 |
| FGK45+IMPRIME vs FGK45+IMPRIME+ T cell depletion | <0.0001 |
| Gemcitabine+IMPRIME vs Gemcitabine+IMPRIME+T cell depletion | 0.0036 |

Statistically significant differences were seen between the following treatment groups: (i) control vs gemcitabine +IMPRIME (p = 0.0002), (ii) gemcitabine vs gemcitabine + IMPRIME (p = 0.0012), (iii) IMPRIME vs gemcitabine + IMPRIME (p = 0.0029), (iv) control vs FGK45 + IMPRIME (p < 0.0001), (v) FGK45 vs FGK45 + IMPRIME (p < 0.0001), (vi) IMPRIME vs FGK45 + IMPRIME (p < 0.0001), and (vii) gemcitabine + IMPRIME vs FGK45 + IMPRIME (p = 0.0048). Together, these data show remarkable therapeutic activity when either gemcitabine or FGK45 is combined with IMPRIME PGG, and that the therapeutic activity of both treatment combinations is dependent on T cells. Overall, a greater response rate (90% versus 50%) and improved overall survival (60% vs 0%) was seen when IMPRIME PGG was combined with FGK45 compared with chemotherapy, respectively.

Combining soluble β-glucan with a CD40 agonist is more efficacious than either treatment alone and produces complete and durable tumor regressions in 60% of mice. In addition, the anti-tumor effect of soluble β-glucan plus a CD40 agonist is dependent on CD4 and CD8 T cells.

### Example 5

**Phagocytic myeloid cells are required for durability of the anti-tumor response of soluble β-glucan in combination with an agonistic CD40 antibody:** The role of distinct myeloid cell subsets for anti-tumor activity induced with IMPRIME PGG and a CD40 agonist. Myeloid-targeting compounds were administered to deplete extratumoral macrophages (i.e. clodronate liposomes), to deplete Ly6C⁺ peripheral blood monocytes (i.e. anti-Ly6C antibodies), to deplete tumor-associated macrophages/extratumoral macrophages (i.e. colony-stimulating factor 1 receptor (CSF1R) inhibitor), and to block CD70 expressed by antigen-presenting cells including macrophages and dendritic cells (anti-CD70 antibodies).

C57BL/6 mice (n=10/group) were implanted subcutaneously with a KPC-derived PDAC cell line. Mice were monitored 2-3 times per week for tumor growth by calipers. When tumors reach approximately 4-5 mm in diameter, mice received the following treatments:
Group 1: Control
Group 2: FGK45 + IMPRIME PGG
Group 3: FGK45 + IMPRIME PGG + clodronate liposomes (Gastroenterology. 2015 Jul; 149(1): 201-210.)
Group 4: FGK45 + IMPRIME PGG + anti-Ly6C (Cancer Discov. 2016 Apr; 6(4): 400-413.)
Group 5: FGK45 + IMPRIME PGG + CSF1R inhibitor
Group 6: FGK45 + IMPRIME PGG + CD70 blocking antibody (Nat Commun. 2012 Jul 10;3:948.)

The compounds were administered using the following dosing schema:
1. FGK45 was administered on day 0 at 0.1 mg by intraperitoneal (i.p.) injection
2. IMPRIME PGG was administered on day 0 at 1.2 mg intravenously (i.v.).
3. Clodronate encapsulated liposomes were administered i.p. at 10 mg/kg on days - 2, 2, 5, and 9.
4. Anti-Ly6C antibodies (Monts1) were administered i.p. at 0.5 mg/dose on days - 1, 0, 1, 5, 9.
5. CSF1R inhibitor (GW2580) was administered daily by oral gavage at 160 mg/kg on days -2 to 2 and days 5 to 9.
6. Anti-CD70 antibodies (clone FR70) was administered i.p. at 0.25 mg/dose on days -1, 0, 1, 5.

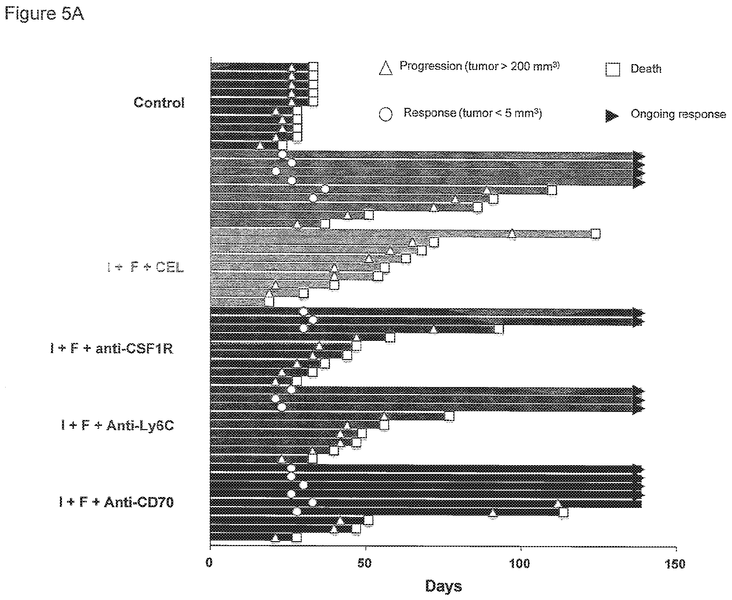

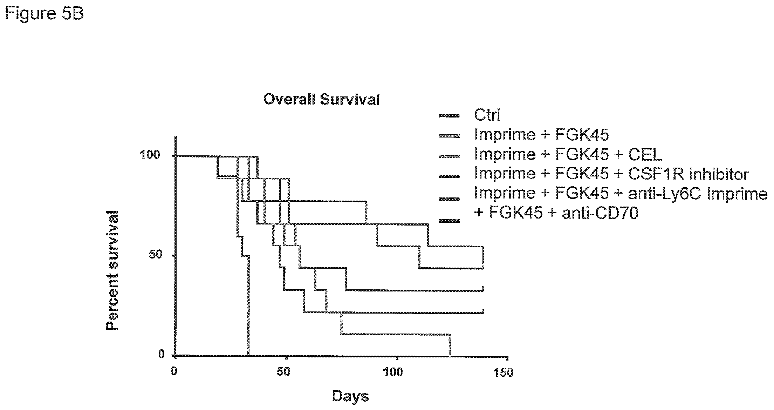

Figures 5 A and 5B show the response to treatment and survival data for each group. The data indicate that phagocytic myeloid cells are required for response durability, because depletion of macrophages by clodronate encapsulated liposomes inhibited the combination therapy.

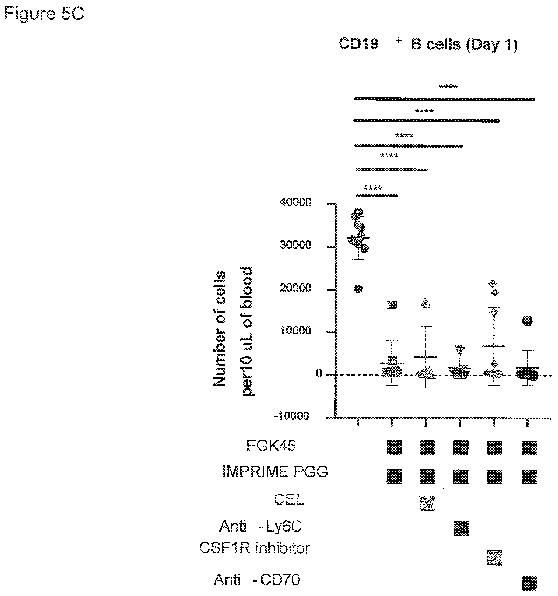

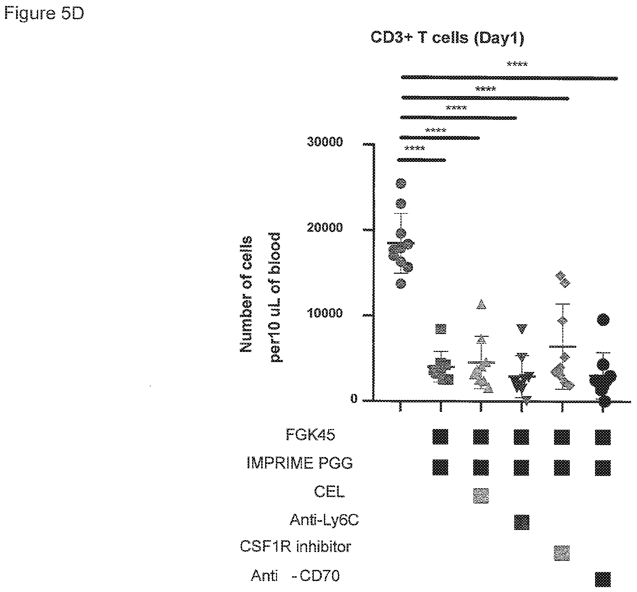

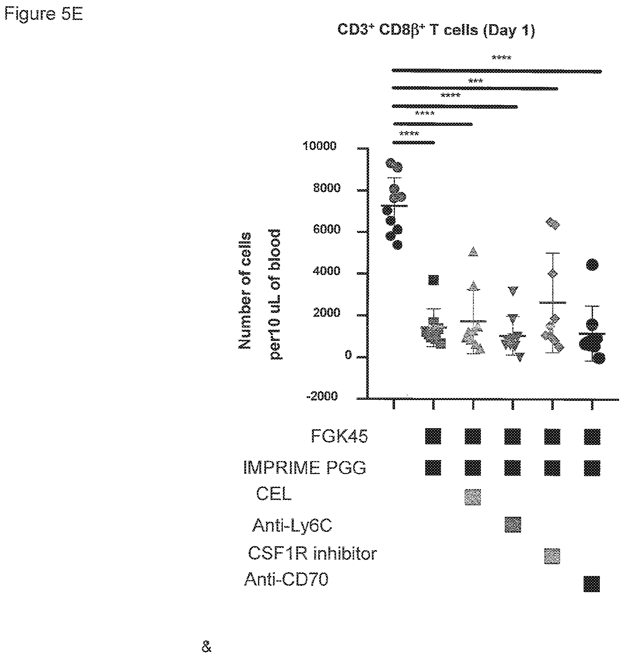

In addition, peripheral blood was collected on each mouse by tail vein bleed on day -2, 0 and 1 of treatment and analyzed by flow cytometry for the presence of CDl9⁺ B cells, CD3⁺ T cells and CD3⁺ CD8β⁺ T cells. The results are shown in Figures 5C, 5D and 5E, and indicate that these cell types leave the peripheral blood circulation.

The synergistic effect that is seen with the combination CD40 agonist/soluble β-glucan therapy is unexpected and remarkable for at least three reasons. First, no chemotherapy is required. As discussed above, the efficacy of CD40 agonists has been reported to be dependent upon the presence of tumor antigen, which is necessary for CD40-activated antigen-presenting cells to induce antigen-specific T cell immunity. Example 2 shows that the combination therapy shows remarkable activity against a poorly immunogenic tumor without chemotherapy. Example 5 shows the requirement of phagocytic myeloid cells suggesting soluble β-glucan alters the phagocytic capacity of tumor-infiltrating myeloid cells and in doing so, improves their capture of tumor antigens. Second, not only is the activity observed with the combination CD40 agonist/soluble β-glucan therapy remarkable without chemotherapy, but it exceeds that seen using chemotherapy followed by a CD40 agonist. Third, it is unexpected and remarkable that the combination of two myeloid targeting agents, a CD40 agonist and soluble β-glucan, resulted in synergistic activity. Example 1 shows that monotherapy with either agent showed limited activity, but the combination CD40 agonist/soluble β-glucan therapy showed significant, more than additive, activity.

The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

Unless otherwise indicated, all numbers expressing quantities of components, molecular weights, and so forth used in the specification and claims are to be understood as being modified in all instances by the term "about." Accordingly, unless otherwise indicated to the contrary, the numerical parameters set forth in the specification and claims are approximations that may vary depending upon the desired properties sought to be obtained by the present invention. At the very least, and not as an attempt to limit the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. All numerical values, however, inherently contain a range necessarily resulting from the standard deviation found in their respective testing measurements.

All headings are for the convenience of the reader and should not be used to limit the meaning of the text that follows the heading, unless so specified.

## Claims

1. Soluble β-glucan for use in a method of treatment of pancreatic cancer, the method comprising administering the soluble β-glucan and an agonistic CD40 antibody, wherein the soluble β-glucan is β(1,6)-[poly-(1,3)-D-glucopyranosyl]-poly-β(1,3)-D-glucopyranose.

2. An agonistic CD40 antibody for use in a method of treatment of pancreatic cancer, the method comprising administering the agonistic CD40 antibody and soluble β-glucan, wherein the soluble β-glucan is β(1,6)-[poly-(1,3)-D-glucopyranosyl]-poly-β(1,3)-D-glucopyranose.

3. Soluble β-glucan and an agonistic CD40 antibody for use in a method of treatment of pancreatic cancer, the method comprising co-administering simultaneously or sequentially the soluble β-glucan and the agonistic CD40 antibody, wherein the soluble β-glucan is β(1,6)-[poly-(1,3)-D-glucopyranosyl]-poly-β(1,3)-D-glucopyranose.

4. The soluble β-glucan for use according to claim 1, the agonistic CD40 antibody for use according to claim 2 or the soluble β-glucan and the agonistic CD40 antibody for use according to claim 3, wherein the pancreatic cancer is a poorly immunogenic pancreatic cancer.

5. The soluble β-glucan for use according to claim 1, the agonistic CD40 antibody for use according to claim 2 or the soluble β-glucan and the agonistic CD40 antibody for use according to claim 3, wherein the soluble β-glucan and the agonistic CD40 antibody are in a single formulation.

6. The soluble β-glucan for use according to claim 1, the agonistic CD40 antibody for use according to claim 2 or the soluble β-glucan and the agonistic CD40 antibody for use according to claim 3, wherein the soluble β-glucan and the agonistic CD40 antibody are in separate formulations.

7. The soluble β-glucan for use according to claim 1, the agonistic CD40 antibody for use according to claim 2 or the soluble β-glucan and the agonistic CD40 antibody for use according to claim 3, wherein the soluble β-glucan is derived from yeast.

8. The soluble β-glucan for use according to claim 1, the agonistic CD40 antibody for use according to claim 2 or the soluble β-glucan and the agonistic CD40 antibody for use according to claim 3, wherein the agonistic CD40 antibody is a non-complement-activating antibody.

9. The soluble β-glucan for use according to claim 1, the agonistic CD40 antibody for use according to claim 2 or the soluble β-glucan and the agonistic CD40 antibody for use according to claim 3, further comprising administering an anti-β-glucan antibody component.

10. The soluble β-glucan for use according to claim 1, the agonistic CD40 antibody for use according to claim 2 or the soluble β-glucan and the agonistic CD40 antibody for use according to claim 3; wherein the soluble β-glucan stimulates a subject's immune system against pancreatic cancer cells.

11. The soluble β-glucan for use, the agonistic CD40 antibody for use, or the soluble β-glucan and the agonistic CD40 antibody for use according to claim 7 wherein the yeast is *Saccharomyces cerevisiae.*

12. The soluble β-glucan for use according to claim 1, the agonistic CD40 antibody for use according to claim 2 or the soluble β-glucan and the agonistic CD40 antibody for use according to claim 3, wherein the agonistic CD40 antibody is an IgG1 or IgG2 antibody.

13. The soluble β-glucan for use according to claim 1, the agonistic CD40 antibody for use according to claim 2 or the soluble β-glucan and the agonistic CD40 antibody for use according to claim 3, wherein the agonistic CD40 antibody is CP-870,893, APX005, ADC-1013, Dacetuzumab, SEA-CD40 or ChiLob 7/4.

## Patentansprüche

1. Lösliches β-Glucan zur Verwendung in einem Verfahren zur Behandlung von Bauchspeicheldrüsenkrebs, wobei das Verfahren das Verabreichen des löslichen β-Glucans und eines agonistischen CD40-Antikörpers umfasst, wobei das lösliche β-Glucan β(1,6)-[Poly-(1,3)-D-glucopyranosyl]-poly-β(1,3)-D-glucopyranose ist.

2. Agonistischer CD40-Antikörper zur Verwendung in einem Verfahren zur Behandlung von Bauchspeicheldrüsenkrebs, wobei das Verfahren das Verabreichen des agonistischen CD40-Antikörpers und von löslichem β-Glucan umfasst, wobei das lösliche β-Glucan β(1,6)-[Poly-(1,3)-D-glucopyranosyl]-poly-β(1,3)-D-glucopyranose ist.

3. Lösliches β-Glucan und ein agonistischer CD40-Antikörper zur Verwendung in einem Verfahren zur Behandlung von Bauchspeicheldrüsenkrebs, wobei das Verfahren die gleichzeitige oder sequentielle CoVerabreichung des löslichen β-Glucans und des agonistischen CD40-Antikörpers umfasst, wobei das lösliche β-Glucan β(1,6)-[Poly-(1,3)-D-glucopyranosyl]-poly-β(1,3)-D-glucopyranose ist.

4. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei der Bauchspeicheldrüsenkrebs ein schwach immunogener Bauchspeicheldrüsenkrebs ist.

5. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei das lösliche β-Glucan und der agonistische CD40-Antikörper in einer einzigen Formulierung vorliegen.

6. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei das lösliche β-Glucan und der agonistische CD40-Antikörper in separaten Formulierungen vorliegen.

7. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei das lösliche β-Glucan von Hefe abgeleitet ist.

8. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei der agonistische CD40-Antikörper ein nicht-komplementaktivierender Antikörper ist.

9. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, ferner umfassend das Verabreichen einer Anti-β-Glucan-Antikörperkomponente.

10. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3; wobei das lösliche β-Glucan das Immunsystem eines Individuums gegen Bauchspeicheldrüsenkrebszellen stimuliert.

11. Lösliches β-Glucan zur Verwendung, der agonistische CD40-Antikörper zur Verwendung oder das lösliche β-Glucan und der agonistische CD40-Antikörper zur Verwendung nach Anspruch 7, wobei die Hefe *Saccharomyces cerevisiae* ist.

12. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei der agonistische CD40-Antikörper ein IgG1- oder IgG2-Antikörper ist.

13. Lösliches β-Glucan zur Verwendung nach Anspruch 1, der agonistische CD40-Antikörper zur Verwendung nach Anspruch 2 oder das lösliche β-Glucan und der agonistische CD40-Antikörper zur Verwendung nach Anspruch 3, wobei der agonistische CD40-Antikörper CP-870,893, APX005, ADC-1013, Dacetuzumab, SEA-CD40 oder ChiLob 7/4 ist.

## Revendications

1. β-glucane soluble destiné à être utilisé dans un procédé de traitement du cancer du pancréas, le procédé comprenant l'administration du β-glucane soluble et d'un anticorps agoniste de CD40, dans lequel le β-glucane soluble est le β(1,6)-[poly-(1,3)-D-glucopyranosyl]-poly-β(1,3)-D-glucopyranose.

2. Anticorps agoniste de CD40 destiné à être utilisé dans un procédé de traitement du cancer du pancréas, le procédé comprenant l'administration de l'anticorps agoniste de CD40 et du β-glucane soluble, dans lequel le β-glucane soluble est le β(1,6)-[poly-(1,3)-D-glucopyranosyl] (1,3)-D-glucopyranose.

3. β-glucane soluble et anticorps agoniste de CD40 destinés à être utilisés dans un procédé de traitement du cancer du pancréas, le procédé comprenant la co-administration simultanée ou séquentielle du β-glucane soluble et de l'anticorps agoniste de CD40, dans lequel le β-glucane soluble est le β(1,6)-[poly-(1,3)-D-glucopyranosyl]-poly-β(1,3)-D-glucopyranose.

4. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, dans lesquels le cancer du pancréas est un cancer du pancréas faiblement immunogène.

5. β-glucane soluble destiné à être utilisé selon la revendication 1, anticorps agoniste de CD40 destiné à être utilisé selon la revendication 2 ou β-glucane soluble et anticorps agoniste de CD40 destiné à être utilisé selon la revendication 3, dans lesquels le β-glucane soluble et l'anticorps agoniste de CD40 sont dans une formulation unique.

6. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, dans lesquels le β-glucane soluble et l'anticorps agoniste de CD40 sont dans des formulations distinctes.

7. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, dans lesquels le β-glucane soluble est dérivé de levure.

8. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, dans lesquels l'anticorps agoniste de CD40 est un anticorps non activant le complément.

9. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, comprenant en outre l'administration d'un composant anticorps anti-β-glucane.

10. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, dans lesquels le β-glucane soluble stimule le système immunitaire d'un sujet contre des cellules cancéreuses pancréatiques.

11. β-glucane soluble destiné à être utilisé, anticorps agoniste de CD40 destiné à être utilisé, ou β-glucane soluble et anticorps agoniste de CD40 destinés à être utilisés selon la revendication 7, dans lesquels la levure est Saccharomyces cerevisiae.

12. β-glucane soluble destiné à être utilisé selon la revendication 1, anticorps agoniste de CD40 destiné à être utilisé selon la revendication 2 ou β-glucane soluble et anticorps agoniste de CD40 destinés à être utilisés selon la revendication 3, dans lesquels l'anticorps agoniste de CD40 est un anticorps IgG1 ou IgG2.

13. β-glucane soluble destiné à être utilisé selon la revendication 1, anticorps agoniste de CD40 destiné à être utilisé selon la revendication 2 ou β-glucane soluble et anticorps agoniste de CD40 destiné à être utilisé
selon la revendication 3, dans lesquels l'anticorps agoniste de CD40 est CP-870,893, APX005, ADC-1013, Dacétuzumab, SEA-CD40 ou ChiLob 7/4.
